# FASCICULE DE BREVET EUROPEEN

(11) **EP 1 354 874 B1**
(45) Date de publication et mention de la délivrance du brevet: **24.11.2004**
(21) Numéro de dépôt: 03290931.9
(22) Date de dépôt: 15.04.2003
(51) Int. Cl.: C07D 209/42, C07C 229/14, C07K 5/02, C07K 5/06

(54) **Nouveau procédé de synthèse de l'acide (2s, 3aS, 7aS)-perhydroindole-2-carboxylique et de ses esters, et application a la synthèse du perindopril**
Verfahren zur Synthese von (2S,3aS,7aS)-Perhydroindol-2-carbonsäure und seiner Estern, und Verwendung in der Synthese von Perindopril
Method for synthesis of (2S,3aS,7aS)-perhydroindole-2-carboxylic acid and esters thereof; and use in the synthesis of perindopril

(43) Date de publication de la demande: 22.10.2003
(73) Titulaire: Les Laboratoires Servier, 92415 Courbevoie Cedex (FR)
(72) Inventeur: Dubuffet, Thierry, 76210 Bolbec (FR); Langlois, Pascal, 76210 Saint Jean de la Neuville (FR)

(56) Documents cités:
- EP-A- 0 308 339

## Description

La présente invention concerne un procédé de synthèse de l'acide (2S, 3aS, 7aS)-perhydroindole-2-carboxylique et de ses esters, et leur application à la synthèse industrielle du perindopril et de ses sels pharmaceutiquement acceptables.

Plus spécifiquement, la présente invention concerne un nouveau procédé de synthèse des dérivés de formule (I) : 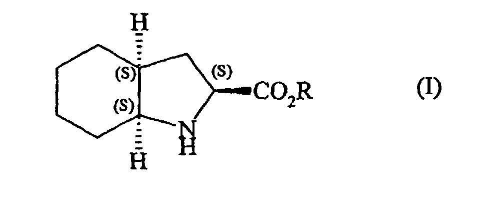 dans laquelle R représente un atome d'hydrogène ou un groupement benzyle ou alkyle (C₁-C₆) linéaire ou ramifié,
ainsi que leurs sels d'addition à un acide ou à une base minéral(e) ou organique.

Les composés de formule (I) obtenus selon le procédé de l'invention sont utiles dans la synthèse du perindopril de formule (II) : 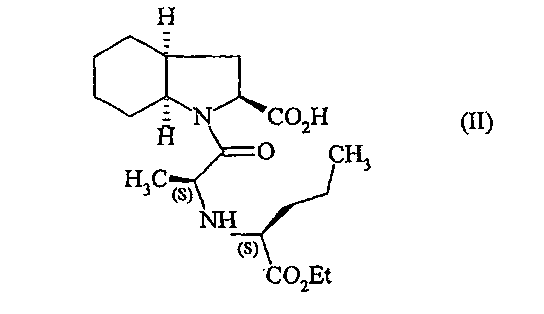 ainsi que dans celle de ses sels pharmaceutiquement acceptables.

Le perindopril, ainsi que ses sels, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse performant, permettant l'obtention sélective du diastéréoisomère (S,S,S) avec un bon rendement et une excellente pureté, à partir de matières premières bon marché.

Quelques méthodes de préparation des composés de formule (I) sont déjà connues.

Ainsi, le brevet EP 0 037 231 utilise comme matière première l'acide indole 2-carboxylique, qui est soumis à une hydrogénation catalytique sur rhodium pour donner un mélange des deux isomères cis endo de configurations respectives (2S, 3aS, 7aS) et (2R, 3aR, 7aR). Ce mélange est ensuite séparé de façon particulièrement laborieuse : synthèse du dérivé N-benzoylé, cristallisation fractionnée du sel du diastéréoisomère avec la (S)-α-phényl-éthylamine, libération des deux dérivés (S, S, S) et (R, R, R) N-benzoylés, puis élimination du groupement benzoyle, suivie d'un passage sur colonne échangeuse d'ions et d'une recristallisation.

Le brevet EP 0 115 345, pour cette même séparation, utilise plusieurs étapes nécessitant l'estérification de la fonction acide carboxylique par l'alcool benzylique, la salification de l'amino ester par la N-benzyloxycarbonyl-(S)-phénylalanine, la séparation par cristallisation fractionnée de l'isomère (S, S, S), la libération de la fonction aminée optionnellement suivie de la libération du groupement acide carboxylique.

Les brevets EP 0 308 339 et EP 0 308 341 utilisent également comme matière première l'acide indole 2-carboxylique, qui est dans un premier temps réduit en acide indoline 2-carboxylique, pour donner un mélange d'acide indoline carboxylique 2R et 2S, lesquels sont ensuite séparés par cristallisation fractionnée. L'isomère 2S est ensuite soumis à hydrogénation catalytique pour conduire au composé de formule (I).

La demanderesse a présentement mis au point un nouveau procédé de synthèse des dérivés de formule (I), à partir d'une matière première particulièrement bon marché, et qui permet l'obtention sélective du diastéréoisomère (S, S, S) avec un bon rendement et une excellente pureté.

Plus spécifiquement, la présente invention concerne un procédé de synthèse des composés de formule (I), caractérisé en ce que l'on condense la (L)-sérine protégée de formule (III) : 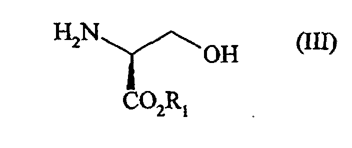 dans laquelle R₁ représente un groupement alkyle (C₁-C₆) linéaire ou ramifié ou benzyle,
avec un composé de formule (IV) : 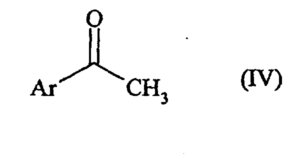 dans laquelle Ar représente, soit un groupement phényle éventuellement substitué par un ou plusieurs groupements alkyle (C₁-C₆) linéaire ou ramifié, soit un groupement naphtyle,
pour conduire au composé de formule (V) : 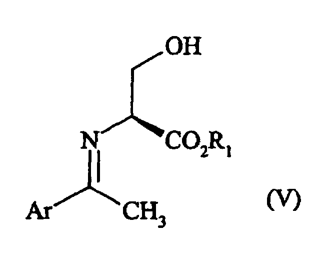 dans laquelle Ar et R₁ sont tels que définis précédemment,
dont on effectue la réduction diastéréosélective, pour conduire au composé de formule (VI): 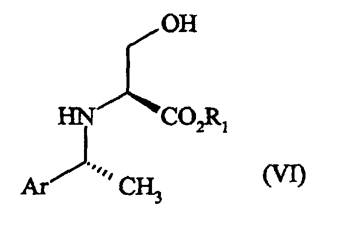 dans laquelle Ar et R₁ sont tels que définis précédemment,
que l'on condense avec la cyclohexanone, pour conduire au composé de formule (VII) : 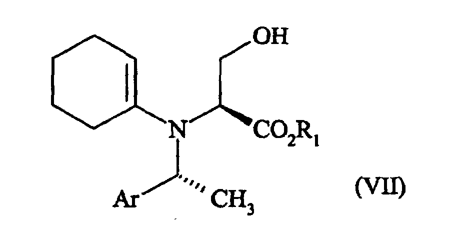 dans laquelle Ar et R₁ sont tels que définis précédemment,
que l'on transforme par halogénation en composé de formule (VIII) : 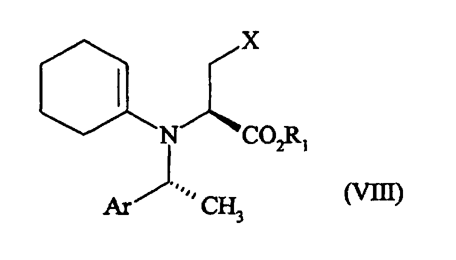 dans laquelle Ar et R₁ sont tels que définis précédemment, et X représente un atome de chlore, de brome ou d'iode,
dont on effectue la cyclisation radicalaire par traitement avec l'hydrure de n-tributylétain, en présence d'un initiateur de radicaux, pour conduire au composé de formule (IX) : 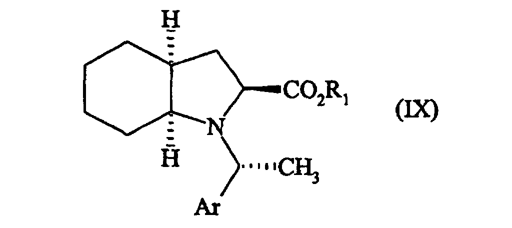 dans laquelle Ar et R₁ sont tels que définis précédemment,
dont on déprotège la fonction amine et, lorsqu'on le souhaite, la fonction acide, pour conduire au composé de formule (I).

Les composés de formules (VII) et (VIII) sont des produits nouveaux, utiles comme intermédiaires de synthèse dans l'industrie chimique ou pharmaceutique, notamment dans la synthèse du composé de formule (I), et font à ce titre partie intégrante de invention.

Les composés préférés de formules (VII) et (VIII) sont ceux pour lesquels R₁ représente le groupement méthyle.

Les composés préférés de formules (VII) et (VIII) sont ceux pour lesquels Ar représente un groupement phényle éventuellement substitué par un groupement méthyle.

Le composé préféré de formule (VIII) est celui pour lequel X représente un atome d'iode.

La réaction de réduction diastéréosélective de l'imine de formule (V) est de façon avantageuse une réaction d'hydrogénation catalytique en phase homogène ou hétérogène. Parmi les catalyseurs utilisables dans la réaction d'hydrogénation en phase hétérogène, on peut citer à titre d'exemple le palladium. Parmi les catalyseurs utilisables dans la réaction d'hydrogénation en phase homogène, on peut citer à titre d'exemple les dérivés du rhodium, préférentiellement les dérivés de rhodium comportant un ligand de type diphosphine, plus préférentiellement les dérivés du rhodium et de l'acide 3-[[(1S,3S)-3-(diphénylphosphino)-1-méthylbutyl]-(phényl)-phosphino]-benzènesulfonique.

Parmi les initiateurs de radicaux utilisables dans la réaction de cyclisation radicalaire, on peut citer à titre non limitatif le 2,2'-azobisisobutyronitrile, le peroxyde de lauroyle et le peroxyde de tert-butyle.
L'initiateur de radicaux préféré est le 2,2'-azobisisobutyronitrile.

Le composé de formule (I) ainsi obtenu a une très bonne pureté chimique et énantiomérique, ce qui rend son emploi particulièrement avantageux dans la synthèse du perindopril de formule (II).

A titre d'illustration, le couplage du composé de formule (I) obtenu selon le procédé de invention avec le composé de formule (X) : 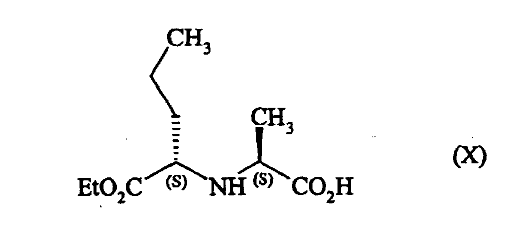 permet d'obtenir le perindopril de formule (II) avec une pureté et un rendement très satisfaisants.

### Abréviations :

### COD : 1,5-cyclooctadiène.

### EXEMPLE : Acide (2S,3aS,7aS)-perhydroindole-2-carboxylique

### Stade A : (2S)-3-Hydroxy-2-{[1-(o-tolyl)-éthylidène]-amino} propanoate de méthyle

Dans un réacteur, chauffer à reflux 200 g de l'ester méthylique de la (L)-sérine, 225 g de 2-méthylacétophénone, 32 g d'acide para-toluènesulfonique et 1 l de toluène en éliminant l'eau formée par entraînement azéotropique. Lorsqu'il ne décante plus d'eau, évaporer le toluène, pour conduire au (2S)-3-hydroxy-2-{[1-(0-tolyl)-éthylidène]-amino} propanoate de méthyle.

### Stade B : (2S)-3-Hydroxy-2-{[(1R)-1-(o-tolyl)-éthyl]-amino} propanoate de méthyle

Dans un autoclave, placer 1,85 g de 3-[[(1S,3S)-3-(diphénylphosphino)-1-méthylbutyl]-(phényl)-phosphino]-benzènesulfonate de sodium, 0,84 g de [Rh(COD)Cl]₂, 1l d'acétate d'éthyle et 1l d'eau, amener à pH=12, puis, après 1/4h d'agitation à température ambiante, charger 200 g du composé obtenu dans le stade précédent et hydrogéner sous pression de 70 bars. Séparer la phase organique, puis la laver, la filtrer et évaporer le solvant, et purifier le résidu obtenu par réempâtage sur silice pour conduire au (2S)-3-hydroxy-2-{[(1R)-1-(o-tolyl)-éthyl]-amino} propanoate de méthyle.

### Stade C : (2S)-2-{1-Cyclohexén-1-yl-3-Hydroxy-[(1R)-1-(o-tolyl)-éthyl]-amino} propanoate de méthyle

Dans un réacteur, chauffer à reflux 200 g du composé obtenu au stade précédent, 83 g de cyclohexanone, 16 g d'acide para-toluènesulfonique et 1 l de toluène en éliminant l'eau formée par entraînement azéotropique. Lorsqu'il ne décante plus d'eau, évaporer le toluène pour conduire au (25)-2-{1-cyclohexén-1-yl-3-hydroxy-[(1R)-1-(o-tolyl)-éthyl]-amino} propanoate de méthyle.

### Stade D : (2S)-2-{3-Iodo-1-cyclohexén-1-yl-[(1R)-1-(o-tolyl)-éthyl]-amino} propanoate de méthyle

Dans un réacteur, charger 248 g de triphénylphosphine, 64 g d'imidazole et 1 l de toluène, puis ajouter à température ambiante une solution de 216 g d'iode dans le toluène. Un précipité jaune se forme. Après 10 mn d'agitation à température ambiante, ajouter 200 g du composé obtenu au stade précédent en solution dans le toluène. Agiter le mélange réactionnel pendant 5 h, puis le verser sur un mélange d'eau et d'éther isopropylique. Laver la phase organique, l'évaporer puis ajouter de l'éther isopropylique et porter au reflux 15 mn. Après retour à température ambiante, le mélange est agité 2 heures, filtré, et le filtrat est évaporé pour conduire au (2S)-2-{3-iodo-1-cyclohexén-1-yl-[(1R)-1-(o-tolyl)-éthyl]-amino}propanoate de méthyle.

### Stade E : (2S, 3aS, 7aS)-1-[(1R)-1-(o-Tolyl)-éthyl]octahydro-1H-indole-2-carboxylate de méthyle

Dans un réacteur, chauffer à reflux 200 g du composé obtenu au stade précédent et 1 l de toluène, puis ajouter en 2 h une solution de 164 g d'hydrure de tri-n-butyl-étain et de 7,7 g d'azobisisobutyronitrile dans le toluène.
Après 2 h supplémentaires de chauffage au reflux, évaporer le toluène, ramener le mélange réactionnel à température ambiante, puis ajouter de l'éther isopropylique et une solution aqueuse à 8 % de fluorure de potassium.
Après 1 h d'agitation à température ambiante, la phase organique est séparée, lavée, séchée puis évaporée, pour conduire au (2S)-1-[(1R)-1-(o-tolyl)-éthyl]octahydro-1H-indale-2-carboxylate de méthyle, sous la forme d'un mélange des composés (3aS,7aS) et (3aR,7aR), duquel l'isomère (3aS,7aS), majoritaire, est isolé par chromatographie sur silice. Le (2S, 3aS,7aS)-1-[(1R)-1-(o-tolyl)-éthyl]octahydro-1H-indole-2-carboxylate de méthyle est ainsi obtenu avec une pureté chimique de 97 % et une pureté énantiomérique de 99 %.

### Stade F : Acide (2S, 3aS, 7aS)-perhydroindole-2-carboxylique

Dans un hydrogénateur, placer 200 g du composé obtenu dans le stade précédent, en solution dans l'éthanol, puis 5 g de Pd/C à 10 %. Hydrogéner à température ambiante jusqu'à absorption de la quantité théorique d'hydrogène. Eliminer le catalyseur par filtration, puis évaporer le solvant et saponifier le résidu obtenu par la soude pour conduire, après isolement et purification, à l'acide (2S, 3aS, 7aS)-perhydroindole 2-carboxylique avec une pureté chimique de 98 % et une pureté énantiomérique de 99 %.

## Revendications

1. Procédé de synthèse des composés de formule (I) : 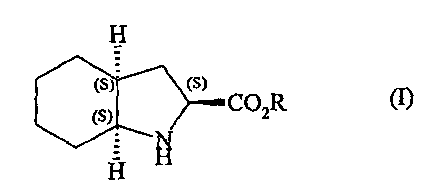 dans laquelle R représente un atome d'hydrogène ou un groupement benzyle ou alkyle (C₁-C₆) linéaire ou ramifié,
**caractérisé en ce que** l'on condense la (L)-sérine protégée de formule (III) : 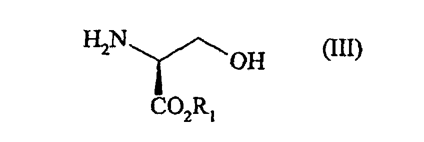 dans laquelle R₁ représente un groupement alkyle (C₁-C₆) linéaire ou ramifié ou benzyle,
avec un composé de formule (IV) : 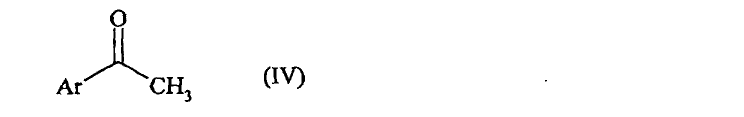 dans laquelle Ar représente, soit un groupement phényle éventuellement substitué par un ou plusieurs groupements alkyle (C₁-C₆) linéaire ou ramifié, soit un groupement naphtyle,
pour conduire au composé de formule (V) : 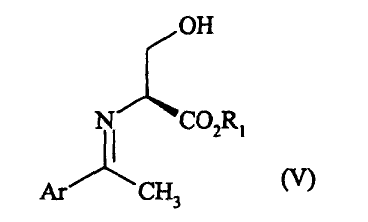 dans laquelle Ar et R₁ sont tels que définis précédemment,
dont on effectue la réduction diastéréosélective, pour conduire au composé de formule (VI) : 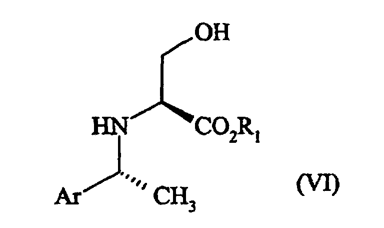 dans laquelle Ar et R₁ sont tels que définis précédemment,
que l'on condense avec la cyclohexanone, pour conduire au composé de formule (VII) : 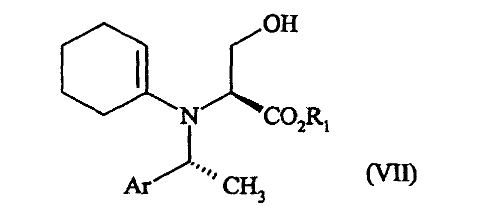 dans laquelle Ar et R₁ sont tels que définis précédemment,
que l'on transforme par halogénation en composé de formule (VIII) : 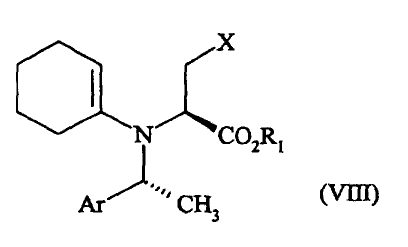 dans laquelle Ar et R₁ sont tels que définis précédemment, et X représente un atome de chlore, de brome ou d'iode,
dont on effectue la cyclisation radicalaire par traitement avec l'hydrure de n-tributylétain, en présence d'un initiateur de radicaux, pour conduire au composé de formule (IX) : 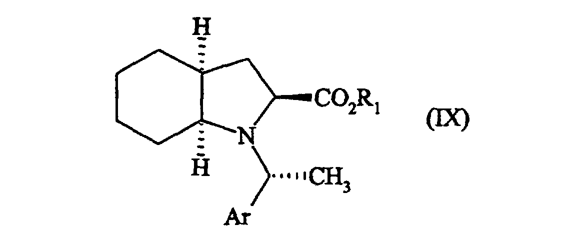 dans laquelle Ar et R₁ sont tels que définis précédemment,
dont on déprotège la fonction amine et, lorsqu'on le souhaite, la fonction acide, pour conduire au composé de formule (I).

2. Procédé de synthèse selon la revendication 1, **caractérisé en ce que** la réaction de réduction diastéréosélective est une réaction d'hydrogénation catalysée par le palladium ou par un dérivé de rhodium.

3. Procédé de synthèse selon la revendication 2, **caractérisé en ce que** la réaction de réduction diastéréosélective est une réduction d'hydrogénation en phase homogène, catalysée par un dérivé du rhodium et de l'acide 3-[[(1S,3S)-3-(diphénylphosphino)-1-méthylbutyl]-(phényl)-phosphino]-benzènesulfonique.

4. Procédé de synthèse selon l'une quelconque des revendications 1 à 3, **caractérisé en ce que** l'initiateur de radicaux est le 2,2'-azobisisobutyronitrile.

5. Composé de formule (VII) : 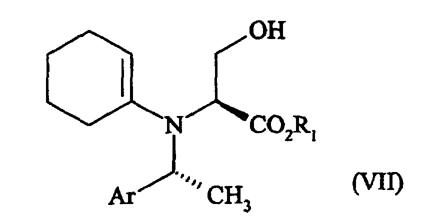 dans laquelle Ar représente, soit un groupement phényle éventuellement substitué par un ou plusieurs groupements alkyle (C₁-C₆) linéaire ou ramifié, soit un groupement naphtyle, et R₁ représente un groupement alkyle (C₁-C₆) linéaire ou ramifié ou benzyle.

6. Composé de formule (VIII) : 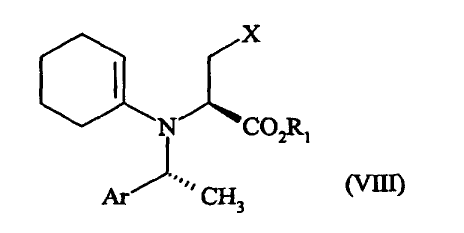 dans laquelle Ar représente, soit un groupement phényle éventuellement substitué par un ou plusieurs groupements alkyle (C₁-C₆) linéaire ou ramifié, soit un groupement naphtyle, R₁ représente un groupement alkyle (C₁-C₆) linéaire ou ramifié ou benzyle, et X représente un atome de chlore, de brome ou d'iode.

7. Composé de formule (VII) selon la revendication 5, **caractérisé en ce que** R₁ représente le groupement méthyle.

8. Composé de formule (VII) selon l'une quelconque des revendications 5 ou 7, **caractérisé en ce que** Ar représente un groupement phényle éventuellement substitué par un groupement méthyle.

9. Composé de formule (VIII) selon la revendication 6, **caractérisé en ce que** R₁ représente le groupement méthyle.

10. Composé de formule (VIII) selon l'une quelconque des revendications 6 ou 9, **caractérisé en ce que** Ar représente un groupement phényle éventuellement substitué par un groupement méthyle.

11. Composé de formule (VIII) selon l'une quelconque des revendications 6, 9 ou 10, **caractérisé en ce que** X représente un atome d'iode.

12. Procédé de synthèse du perindopril ou de ses sels pharmaceutiquement acceptables à partir d'un composé de formule (I), **caractérisé en ce que** ledit composé de formule (I) est obtenu selon le procédé de la revendication 1.

## Patentansprüche

1. Verfahren zur Synthese der Verbindungen der Formel (I): 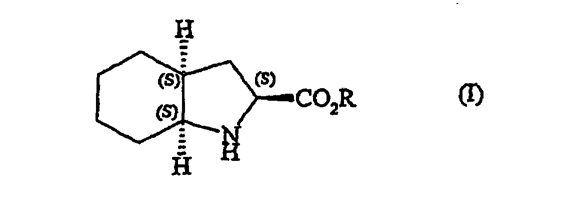 in der R ein Wasserstoffatom oder eine Benzylgruppe oder eine geradkettige oder verzweigte (C₁-C₆)-Alkylgruppe bedeutet,
**dadurch gekennzeichnet, daß** man geschütztes (L)-Serin der Formel (III): 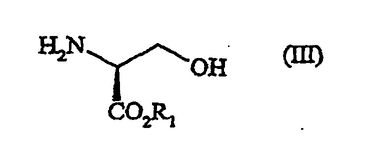 in der R₁ eine geradkettige oder verzweigte (C₁-C₆)-Alkylgruppe oder eine Benzylgruppe bedeutet,
mit einer Verbindung der Formel (IV): 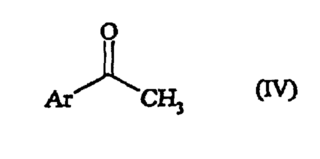 in der Ar entweder eine gegebenenfalls durch eine oder mehrere geradkettige oder verzweigte (C₁-C₆)-Alkylgruppen substituierte Phenylgruppe oder eine Naphthylgruppe bedeutet, kondensiert zur Bildung der Verbindung der Formel (V): 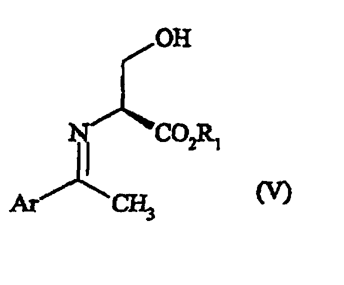 in der Ar und R₁ die oben angegebenen Bedeutungen besitzen,
welche man einer diastereoselektiven Reduktion unterwirft zur Bildung der Verbindung der Formel (VI): 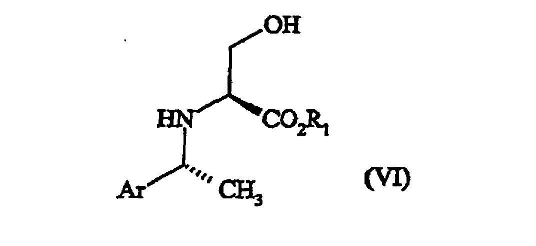 in der Ar und R₁ die oben angegebenen Bedeutungen besitzen,
welche man mit Cyclohexanon kondensiert zur Bildung der Verbindung der Formel (VII): 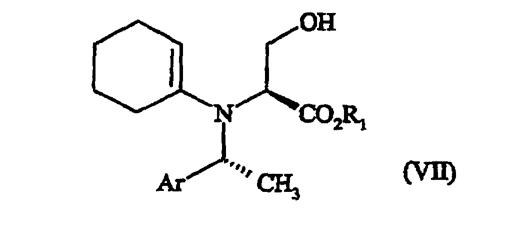 in der Ar und R₁ die oben angegebenen Bedeutungen besitzen,
welche man durch Halogenierung in die Verbindung der Formel (VIII) überführt: 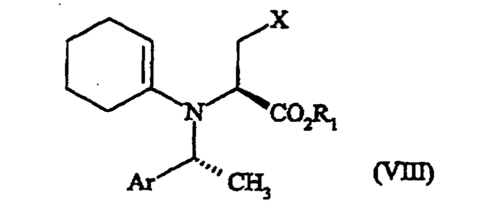 in der Ar und R₁ die oben angegebenen Bedeutungen besitzen und X ein Chlor-, Brom- oder Iodatom darstellt,
welche man in Gegenwart eines Radikalinitiators durch Behandeln mit n-Tributylzinnhydrid einer radikalischen Cyclisierung unterzieht zur Bildung der Verbindung der Formel (IX): 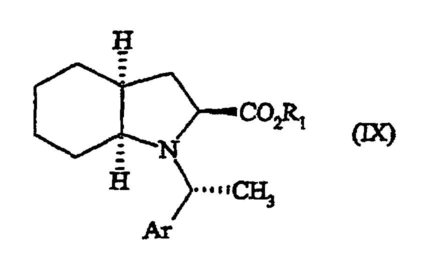 in der Ar und R₁ die oben angegebenen Bedeutungen besitzen,
von welcher man die Schutzgruppe der Aminfunktion und gewünschtenfalls der Säurefunktion abspaltet zur Bildung der Verbindung der Formel (I).

2. Syntheseverfahren nach Anspruch 1, **dadurch gekennzeichnet, daß** die diastereoselektive Reduktion eine Hydrierungsreaktion ist, die durch Palladium oder ein Rhodiumderivat katalysiert wird.

3. Syntheseverfahren nach Anspruch 2, **dadurch gekennzeichnet, daß** die diastereoselektive Reduktion eine Hydrierungsreaktion in homogener Phase ist, die durch ein Rhodiumderivat und 3-[[(1S,3S)-3-(Diphenylphosphino)-1-methylbutyl]-(phenyl)-phosphino]-benzolsulfonsäure katalysiert wird.

4. Syntheseverfahren nach einem der Ansprüche 1 bis 3, **dadurch gekennzeichnet, daß** der Radikalinitiator 2,2'-Azobisisobutylnitril ist.

5. Verbindung der Formel (VII): 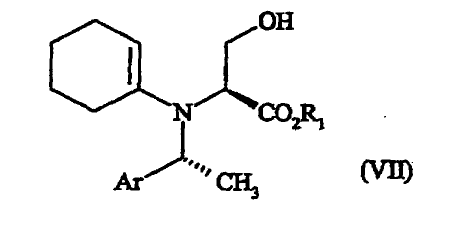 in der Ar entweder eine gegebenenfalls durch eine oder mehrere geradkettige oder verzweigte (C₁-C₆)-Alkylgruppen substituierte Phenylgruppe oder eine Naphthylgruppe bedeutet und R₁ eine geradkettige oder verzweigte (C₁-C₆)-Alkylgruppe oder eine Benzylgruppe darstellt.

6. Verbindung der Formel (VIII): 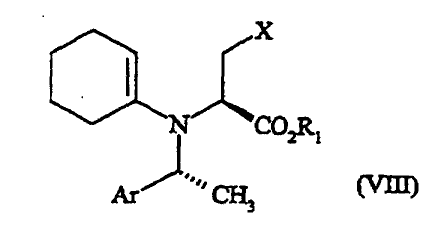 in der Ar entweder eine gegebenenfalls durch eine oder mehrere geradkettige oder verzweigte (C₁-C₆)-Alkylgruppen substituierte Phenylgruppe oder eine Naphthylgruppe bedeutet, R₁ eine geradkettige oder verzweigte (C₁-C₆)-Alkylgruppe oder eine Benzylgruppe darstellt und X ein Chlor-, Brom- oder Iodatom bedeutet.

7. Verbindung der Formel (VII) nach Anspruch 5, **dadurch gekennzeichnet, daß** R₁ die Methylgruppe darstellt.

8. Verbindung der Formel (VII) nach einem der Ansprüche 5 oder 7, **dadurch gekennzeichnet, daß** Ar eine gegebenenfalls durch eine Methylgruppe substituierte Phenylgruppe bedeutet.

9. Verbindung der Formel (VIII) nach Anspruch 6, **dadurch gekennzeichnet, daß** R₁ eine Methylgruppe darstellt.

10. Verbindung der Formel (VIII) nach einem der Ansprüche 6 oder 9, **dadurch gekennzeichnet, daß** Ar eine gegebenenfalls durch eine Methylgruppe substituierte Phenylgruppe bedeutet.

11. Verbindung der Formel (VIII) nach einem der Ansprüche 6, 9 oder 10, **dadurch gekennzeichnet, daß** X ein Iodatom bedeutet.

12. Verfahren zur Synthese von Perindopril oder von seinen pharmazeutisch annehmbaren Salzen, ausgehend von einer Verbindung der Formel (I), **dadurch gekennzeichnet, daß** die Verbindung der Formel (I) nach dem Verfahren des Anspruchs 1 hergestellt wird.

## Claims

1. Process for the synthesis of compounds of formula (I) : 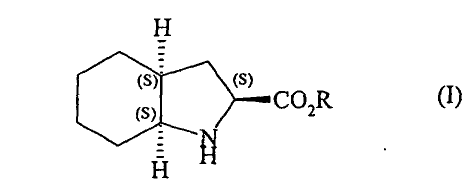 wherein R represents a hydrogen atom or a benzyl or linear or branched (C₁-C₆)alkyl group,
**characterised in that** protected (L)-serine of formula (III) : 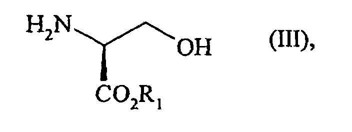 wherein R₁ represents a linear or branched (C₁-C₆)alkyl or benzyl group,
is condensed with a compound of formula (IV) : 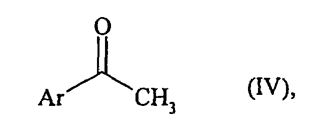 wherein Ar represents either a phenyl group optionally substituted by one or more linear or branched (C₁-C₆)alkyl groups, or a naphthyl group,
to yield a compound of formula (V) : 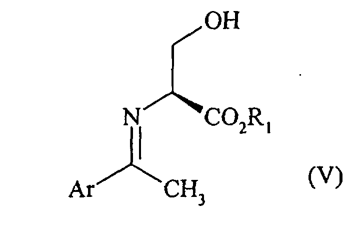 wherein Ar and R₁ are as defined hereinbefore,
which is subjected to diastereoselective reduction to yield a compound of formula (VI) : 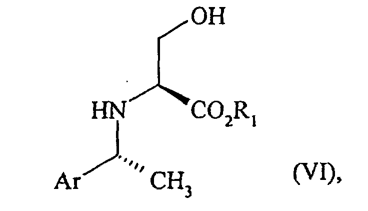 wherein Ar and R₁ are as defined hereinbefore,
which is condensed with cyclohexanone to yield a compound of formula (VII) : 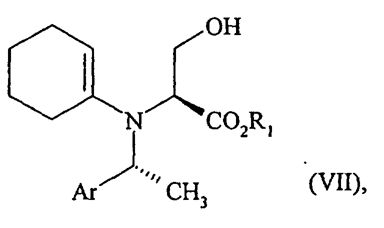 wherein Ar and R₁ are as defined hereinbefore,
which is converted by halogenation to a compound of formula (VIII) : 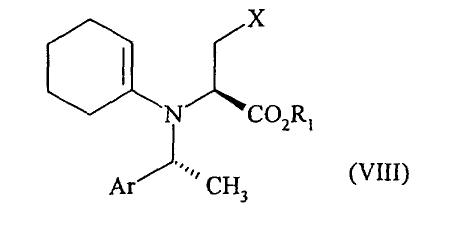 wherein Ar and R₁ are as defined hereinbefore and X represents a chlorine, bromine or iodine atom,
which is subjected to free-radical cyclisation by treatment with n-tributyltin hydride, in the presence of a free-radical initiator, to yield a compound of formula (IX) : 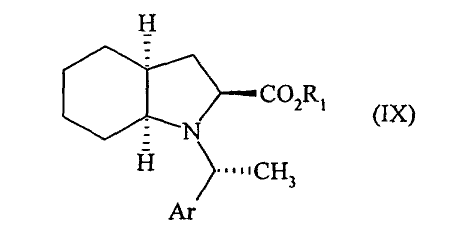 wherein Ar and R₁ are as defined hereinbefore,
the amine function and, if desired, the acid function of which are deprotected to yield a compound of formula (I).

2. Synthesis process according to claim 1, **characterised in that** the diastereoselective reduction reaction is a hydrogenation reaction catalysed by palladium or by a rhodium product.

3. Synthesis process according to claim 2, **characterised in that** the diastereoselective reduction reaction is a hydrogenation reduction in homogeneous phase catalysed by a product derived from rhodium and from 3-[[(1S,3S)-3-(diphenylphosphino)-1-methylbutyl]-(phenyl)-phosphino]-benzenesulphonic acid.

4. Synthesis process according to any one of claims 1 to 3, **characterised in that** the free-radical initiator is 2,2'-azobisisobutyronitrile.

5. Compound of formula (VII) : 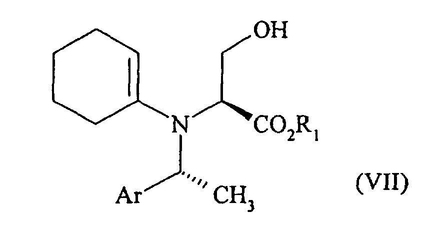 wherein Ar represents either a phenyl group optionally substituted by one or more linear or branched (C₁-C₆)alkyl groups, or a naphthyl group, and R₁ represents a linear or branched (C₁-C₆)alkyl or benzyl group.

6. Compound of formula (VIII) : 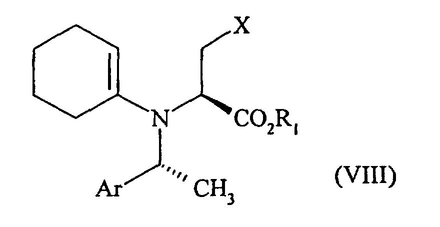 wherein Ar represents either a phenyl group optionally substituted by one or more linear or branched (C₁-C₆)alkyl groups, or a naphthyl group, R₁ represents a linear or branched (C₁-C₆)alkyl or benzyl group, and X represents a chlorine, bromine or iodine atom.

7. Compound of formula (VII) according to claim 5, **characterised in that** R₁ represents a methyl group.

8. Compound of formula (VII) according to claim 5 or claim 7, **characterised in that** Ar represents a phenyl group optionally substituted by a methyl group.

9. Compound of formula (VIII) according to claim 6, **characterised in that** R₁ represents a methyl group.

10. Compound of formula (VIII) according to either claim 6 or claim 9, **characterised in that** Ar represents a phenyl group optionally substituted by a methyl group.

11. Compound of formula (VIII) according to any one of claims 6, 9 and 10, **characterised in that** X represents an iodine atom.

12. Process for the synthesis of perindopril or pharmaceutically acceptable salts thereof starting from a compound of formula (I), **characterised in that** the said compound of formula (I) is obtained according to the process of claim 1.
